# EUROPÄISCHE PATENTANMELDUNG

(11) **EP 0 754 472 A2**
(43) Veröffentlichungstag der Anmeldung: **22.01.1997**
(21) Anmeldenummer: 96108068.6
(22) Anmeldetag: 21.05.1996
(51) Int. Cl.: A61N 5/10, A61M 1/00

(54) **Verfahren zur Herstellung eines flexiblen Fixationsmaterials mit Kathetern oder Schläuchen für die Strahlentherapie**

(30) Priorität: 21.07.1995 DE 19526690
(71) Anmelder: HÜLS AKTIENGESELLSCHAFT, 45764 Marl (DE)
(72) Erfinder: Anders, Christine, Dr., 45721 Haltern (DE); Brathun, Reinhold, 45721 Haltern (DE)

(57) **Zusammenfassung**

Bekanntes Fixiermaterial für die Strahlentherapie wird mühselig mit der Hand für jeden Einzelfall hergerichtet. Auch die Befestigungsmöglichkeiten dieses Materials für das Körpergewebe sind noch nicht zufriedenstellend.

Das erfindungsgemäße Verfahren erlaubt ein bequemes und schnelles Herstellen von Fixationsmaterial, welches Katheter oder Schläuche und medizinische Anbindungen enthält.

## Beschreibung

Für die Strahlenbehandlung von Tumoren in Körperhöhlen, z. B. im Rachen, Darm oder Vaginalbereich sowie in operativ eröffneten Körperbereichen, ist die Brachytherapie mit Kathetern eine anerkannte Methode, bei der die Strahlenquelle (-quellen), gesteuert durch ein Afterloading-Gerät, präzise an den Behandlungsort im Körper gebracht und so bewegt werden kann, daß sich ein vorausberechneter Strahlendosis-Verlauf über der Behandlungsstelle ergibt, so beschrieben von D. E. Wazer, R. Schmidt-Ullrich, W. Chasin, A. Wu, M. Buscher in Am. J. Otolaryngology 10 (3), (1989), 173, und von R. Stepan, P. Lukas, U. Fink, P. Knetschaurek, Ir. Siewert, M. Molls in «Intraoperative Radiotherapy with High Dose Afterloading (Flabs Method) in Colorectal Carcinom» (in F. W. Schildberg, N. Willich, H.-J. Krämling, «Intraoperative Radiation Therapy», Proceedings 4th International Symposium IORT, Munich 1992, Verlag Blaue Eule, Essen).

Um Schäden bei den Patienten zu vermeiden und eine exakte Bestrahlungsplanung sicherzustellen, müssen die Katheter genau positioniert und am oder im Körper fixiert werden. Nur wenn dieses gewährleistet ist, kann die Programmierung des erforderlichen Isodosen-Verlaufs vorgenommen werden und die geplante Bestrahlung mit der notwendigen Sicherheit und Präzision erfolgen.

Übliche Praxis zur Herstellung von fixierten Katheter-Sätzen ist, Gummi-ähnliche Standard-Flachblöcke (1 bis 3 cm hoch, Fläche: 30 x 30 cm; Bezugsquellen sind z. B. Quandt Medizintechnik, Hamburg, oder Mick Radio Nuclear Instruments Inc. Bronx, New York) zurechtzuscheiden und anschließend von Hand mit einer Hohlnadel zu durchbohren, um sie dann mit den Kathetern auszurüsten. Dieses Verfahren hat den Nachteil, daß aufgrund der Reibung und Haftung der Nadelaußenwand mit dem flexiblen Material der Gummi-artige Kunststoff-Körper sich deformiert und die Parallelität der Kanäle verloren geht.

Darüber hinaus lassen sich diese sog. Flabs nicht sicher am Applikationsort fixieren. Es kommt häufig zu Einrissen in den Durchstichen der Fixierfäden und so zur Verschiebung bis gänzlichen Ablösung der Flabs.

Vor diesem Hintergrund war die Erfindung eines mit Kathetern ausgerüsteten Flabs gemäß US-Patentschrift 4 963 128 ein Fortschritt, der aber den Praxisanforderungen noch nicht in vollem Umfang genügt.

Kneschaurek, Wehrmann, Hugo, Stepan Lukas und Molls beschreiben in Strahlentherapie und Onkologie 171 (1995), S. 61, einen Silikongußapplikator, bei dem mit Kunststoff-Hohlnadeln gearbeitet wird, die mit Hilfe einer speziellen Vorrichtung parallel zueinander in der Mittenebene des Flabs eingestochen werden. Allerdings berichten die Verfasser über Schwierigkeiten beim Einbringen der Nadeln, so daß häufig Silikonguß-Körper verworfen werden müssen.

Zylindrische Kunststoff-Katheter-Systeme stehen ebenfalls nicht zur Verfügung. Daher behilft man sich mit selbst durchbohrten Kunststoffteilen, so beschrieben in der oben zitierten Publikation von D. E. Wazer et al., S. 177.

Aufgabe dieser Erfindung war daher, ein Verfahren zur Herstellung flexibler Fixationsformkörper für Katheter oder Schläuche bereitzustellen, das für die Strahlentherapie, insbesondere für die Brachytherapie, geeignete Applikationskörper mit korrekt parallel laufenden Kathetern bereitstellt. Die gestellte Aufgabe wird erfindungsgemäß entsprechend den Angaben der Patentansprüche gelöst. Dabei war es erforderlich, daß der die Katheter umgebende Kunststoff weich und flexibel ist, das Kunststoff-Material Dichten von 0,9 bis 1,2 g/cm³ besitzt und gemessen an der Applikationsdauer und Schwere der Indikation nicht gesundheitsschädigend ist sowie nach den üblichen Verfahren einer Sterilisation unterzogen werden kann. Weiterhin kennzeichnend für die vorliegende Erfindung ist die Ausrüstung dieser Kunststoff-Katheter-Systeme mit einem unter der Oberfläche eingearbeiteten, nicht-fasernden, körperfreundlichen Kunststoffaser-Gewebe über die gesamte Fläche oder einem koaxialen Kunststoffaser-Gewebe am unteren Teil der zylindrischen Körper, in allen Fallen auf der von der Bestrahlungsseite abgewandten Seite oder abseits von dem Bestrahlungsbereich. Somit sind die erfindungsgemäßen Katheter-Kunststoff-Systeme beliebig zuschneidbar, und sie können in beliebiger Weise und an jeder Stelle sicher sowie reißfest fixiert werden.

Als Kunststoff-Materialien zur Führung und Fixierung von der Afterloading-Kathetern sind körperfreundliche, weiche Kunststoffe auf Basis von Polyurethanen, Polyolefinen, Polycarbonaten, Polyvinylchlorid, Polysulfonen, Polyethern, Polyestern, Polyamiden, Polyacrylaten, Silikongummi sowie -Kautschuken und analogen Polymeren mit und ohne Weichmacher einsetzbar, deren Dichten im Bereich von 0,8 bis 1,5 g/cm³, eine möglichst geringe γ-Strahlenabsorption besitzen, transparent oder zumindest transluzend sind und ohne Bildung schädlicher Zersetzungsprodukte nach gängigen Verfahren sterilisierbar sind. Grundsätzlich sind auch Gel-artige Polymersysteme geeignet.

Zur Herstellung der erfindungsgemäßen flachen, quadratischen oder rechteckigen Applikatoren werden die Katheter oder Schläuche durch die jeweils gegebenüberliegenden Bohrungen der Gieß- oder Gelierformen, die aus Glas, chemisch nicht angreifbarem oder oberflächevergütetem Metall - wie z. B. vergütet mit polierten Gold-Platinen -, emaillierten Metallen oder glasierten Keramiken oder auch hochwertigem Graphit bestehen können, hindurchgezogen und gespannt. Wesentlich für die Auswahl des Materials für die Gieß- oder Gelierform ist, daß es bei den Verarbeitungstemperaturen formstabil ist und weder chemische Reaktionen mit dem Gieß- und Gelier-Medium eingeht, die zu Anbackungen oder gewebeschädigenden, toxischen oder krebserzeugenden Stoffen führen, noch ebenso schädliche Stoffe aus seinem Inneren an das Kunststoff-Katheter-System abgibt. Weiterhin müssen die Gieß- und Gelierformen gut zu reinigen sein. Vorteilhaft wäre auch ihre Sterilisierbarkeit für spezielle Anforderungen.

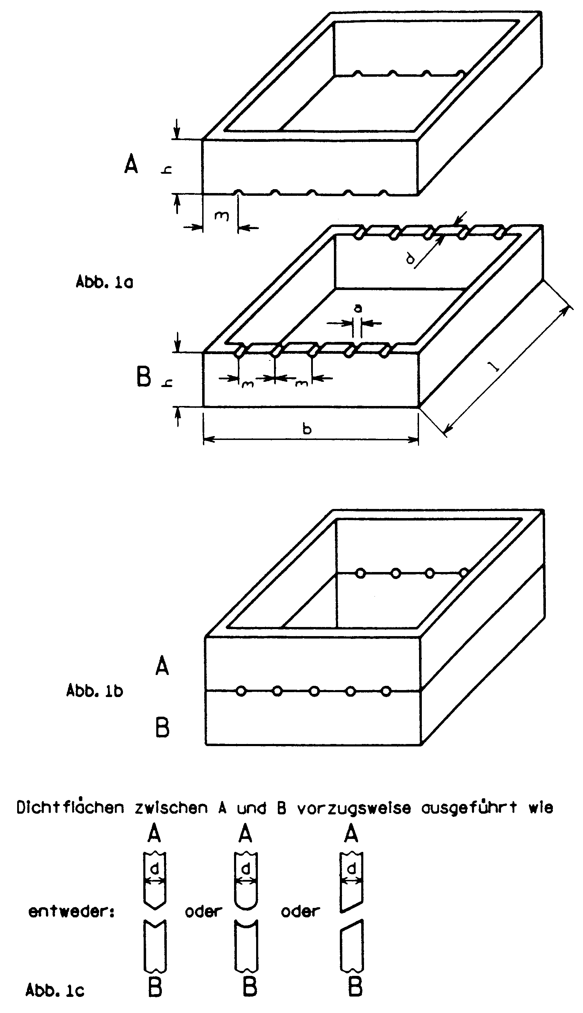

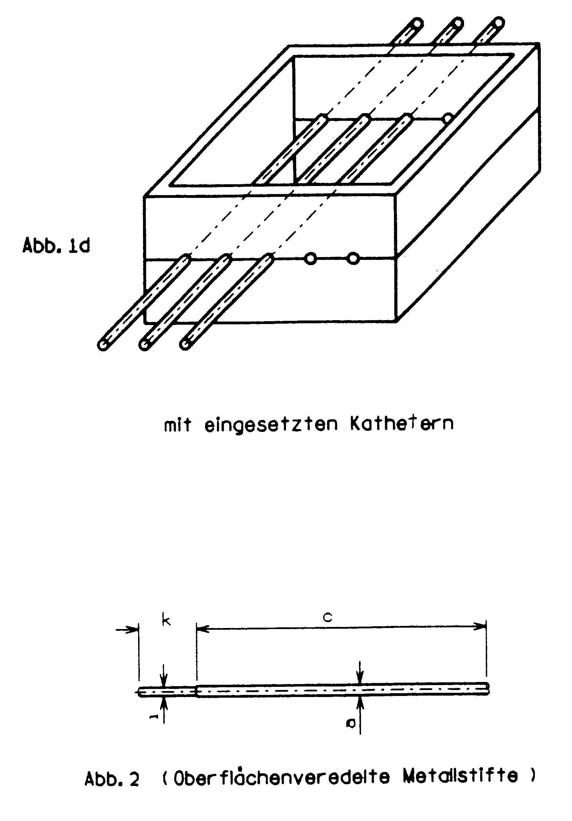

Die Dichtflächen der Gieß- bzw. Gelierformteile (Abb. 1a, 1c) sind vorzugsweise schräg, rund oder konisch zu gestalten, um sicheres Zusammensetzen und hohe Dichtigkeit zu erreichen.

Nach Einspannen der Katheter und Zusammensetzen und gegebenenfalls Zusammenklammern der Form wird das lufthärtende oder Initiator härtende oder gelierende flüssige Kunststoffgut in die Form gegossen, bis die Flüssigkeit auf einer bestimmten Höhe über den eingespannten Kathetern steht. Nach kurzem Anhärten bzw. Angelieren wird das Kunststoff-Gewebe eingelegt und die restliche Menge flüssigen Ausgangsmaterials bis zur Sollhöhe aufgegeben. Dem schließt sich die endgültige Aushärtung bzw. Gelierung des Kunststoff-Materials an. Die Ausgelierung der Paste erfolgt in Abhängigkeit des verwendeten Polymers.

Im Falle großer Bohrungen für die Katheter kann hinreichende Dichtigkeit gegenüber der Gieß- oder Gelierflüssigkeit durch Verspachtelung mit teilausgehärtetem oder angeliertem Material erreicht werden.

Muß das flüssige Gieß- oder Geliergut bei höheren Temperaturen verarbeitet werden, die für die eingespannten Katheter unzulässig sind, werden in oben beschriebener Weise oberflächenvergütete Metallstifte eingesetzt, die dem Durchmesser der Katheter entsprechen und an einem Ende mit einem Paßstück versehen sind (Abb. 2), so daß der entsprechende Kunststoff-Katheter ohne Änderung des äußeren Durchmessers angekoppelt werden kann. Nach dem Aushärten oder Gelieren der Kunststoffmasse lassen sich so die Katheter mit Hilfe der Metallstifte durch den Kunststoffkörper hindurchziehen. Vorzugsweise erfolgt ein solcher Vorgang noch im Zustand unvollständiger Aushärtung oder Gelierung und in der Gieß- oder Gelierform, so daß sich das härtende oder gelierende Kunststoffmaterial an die äußeren Katheter-Flächen anschmiegt.

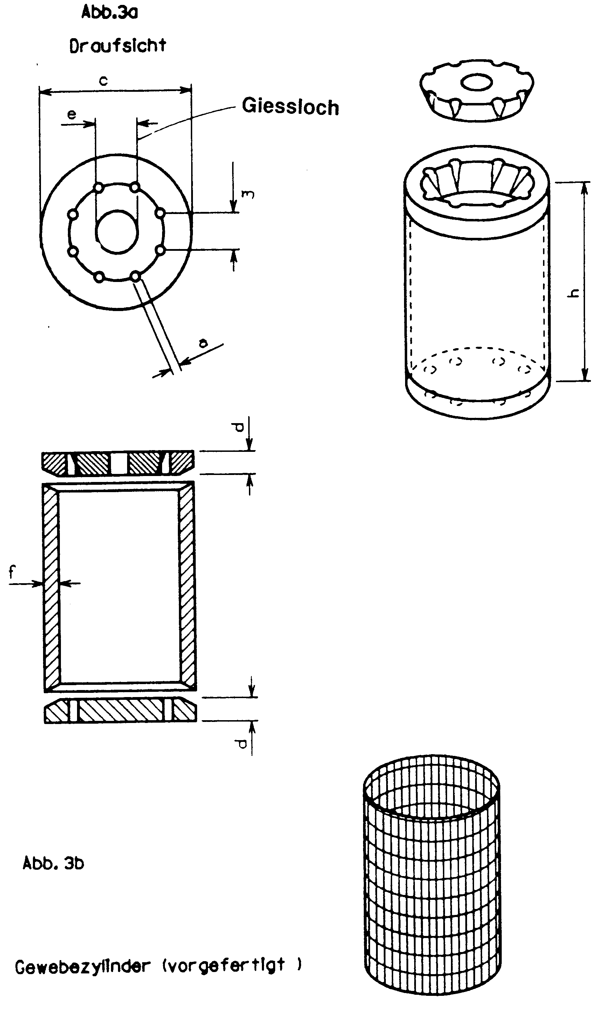

Um bei zylindrischen Gieß- und Gelierformen nach dem Aushärten den fertigen Applikator problemlos zu entfernen, muß der zylindrische Formteil aus zwei Hälften bestehen, die ebenfalls dicht zusammengefügt werden können (Abb. 3). Deckel und Boden setzen sich aus je zwei Ringhälften und Mittelstücken zusammen, wobei nur das obere Zentralstück mit einem Gießloch ausgestattet ist. Sämtliche Teile sind vorzugsweise mit konischen Dichtflächen ausgerüstet, die durch paßgerechtes Schleifen und im Falle von Metallen durch Oberflächenvergütungen an die Sterilitätsanforderungen angepaßt sind.

Die zylindrischen Formen sitzen entweder in einem entsprechenden Ofen oder werden durch eine Spannvorrichtung zusammengehalten, die mit Metallspiralfedern ausgerüstet ist, um sich an die thermische Ausdehnung anzupassen.

Der Gewebeteil ist als Zylinder mit Abmessungen gemäß Anforderungen vorgefertigt (Abb. 3) und wird mit den Kathetern oder oberflächenvergüteten Stiften vor dem Gießen in die Form eingesetzt.

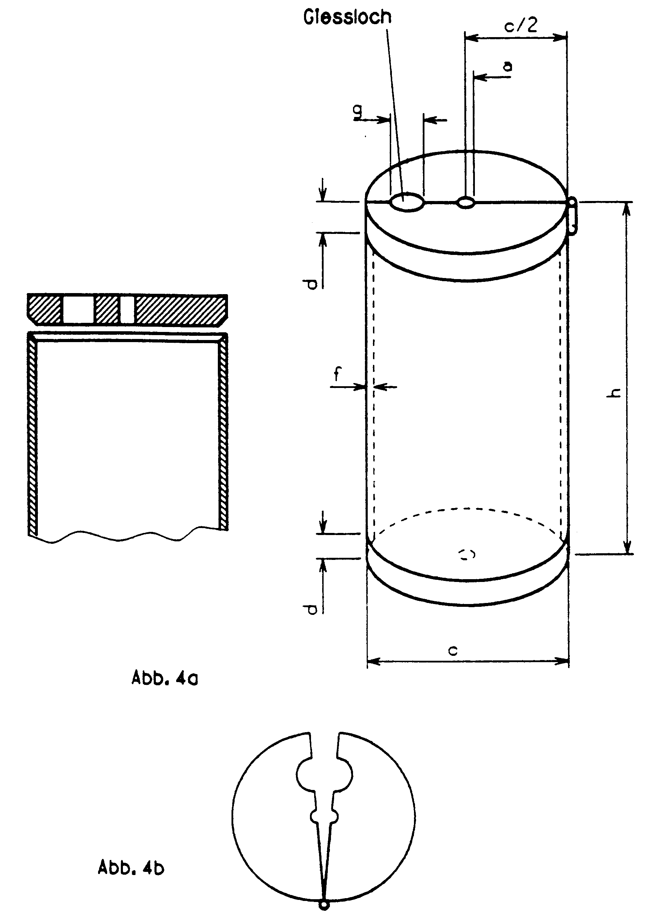

Schlanke zylindrische Formen, die nur mit einem Einzelkatheter auszurüsten sind, gestatten das Herausziehen des fertigen Applikators und sind somit einfacher gebaut (Abb. 4). Nur einer der Deckel besteht aus zwei Teilen.

Die Abmessungen der Abildungen sind nur beispielhaft und nicht auf diese beschränkt. Es können Kunststoff-Katheter-Systeme nach den erfindungsgemäßen Verfahren in jeder gewünschten Größe hergestellt werden.

### Beispiele:

Fließfähige Pasten aus z. B. einzelnen oder mehreren verpastbaren Polyvinylchlorid-Polymerisaten oder Polyacrylaten mit Weichmachern oder Weichmacher-Gemischen erhält man durch Homogenisieren in Dissolver- oder Planetenmischern. Es können Homo-, Co- oder auch Terpolymere eingesetzt werden.

Zur Viskositätsregulierung und Thixotropierung lassen sich kolloidale Kieselsäuren, Metallseifen, Glyzerinmono-oleat und/oder Aluminium- oder/auch Magnesiumsilicate verwenden. Dieses ist insbesondere erforderlich um bei Weichmachergehalten über 50 % die Dispersionen zu stabilisieren. Thixotropierungsmittel werden 0,01 bis 5 Teile auf 100 Teile Polyvinylchlorid oder Polyacrylat eingemischt.

Die Dichten der Kunststoff-Katheter-Systeme können durch Variation der Weichmacher/Polymer-Verhältnisse eingestellt werden.

### Herstellung eines Polyvinylchlorid-Gels:

Nach Vorlage von 10 bis 50 % des Weichmachers (z. B. Edenol, Di(ethylhexyl)phthalat) oder des Weichmachergemisches werden das gesamte verpastbare Polyvinylchlorid in einem Planetenmischer bis zu einer Temperatur von 30 °C hinzugemischt. Anschließend erfolgt eine portionsweise Zugabe der restlichen Weichmacher-Anteile. Zu dieser Masse werden portionsweise insgesamt 2 Teile Glyzerinmonooleat als Thixotropierungsmittel eingearbeitet.

Die erhaltene Gelierungsmasse wird in die Gelierungsform aus Glas, die in diesem Fall anstelle der Katheter mit den oben beschriebenen Metallstiften versehen ist, so eingegeben, daß die flüssige Masse 3 mm über den eingesetzten Stiften steht. Nach 3minütiger Angelierung bei 180 °C wird da Heizaggregat entfernt und nach Abkühlen das Kunststoff-Gewebe eingelegt. Anschließend wird die restliche flüssige Masse hinzugefügt und 15 min. bei 180 °C angeliert.

Nach Abkühlen auf 120 °C können die Katheter, die sich auf den Metallstiften außerhalb des Gelierofens befinden, ohne Mühe und ohne Schaden für die Katheter durch den gelierten Kunststoff hindurchgeschoben werden, wobei die Metallstifte als sichere, maßgerechte Führung für die Katheter dienen.

Nach dem Abkühlen lassen sich die Teile der Gelierform leicht und ohne Kleben der gelierten Kunststoffmasse entfernen. Eine Schrumpfung des Kunststoff-Katheter-Körpers ist praktisch nicht feststellbar.

Nach Entnahme des Kunststoff-Katheter-Systems wird die äußere Oberfläche mit Maisstärke gepudert, wodurch eine weiche, gewebefreundliche und nicht-klebende Oberfläche erhalten wird. Die Dichte des auf diese Weise hergestellten Katheters liegt bei 1,15 g/cm³.

Dieser Applikator kann anschließend auf den geometrischen Flächenbedarf zugeschnitten werden.

### Herstellung eines weichen Polyacrylsäureester-Applikators:

Die Vermischung erfolgt zunächst in gleicher Weise wie bei dem Beispiel für Polyvinylchlorid, mit dem Unterschied, daß 1 % Härter (z. B. Wasserstoffperoxid, Kaliumperoxodisulfat) hinzugefügt werden. Die Aushärtung wird bei 120 °C durchgeführt. Unter diesen Bedingungen bedarf es nicht der vergüteten Metallstifte, so daß die Katheter vor dem Gieß- und Geliervorgang eingesetzt werden können.

## Patentansprüche

1. Verfahren zur Herstellung von flexiblen Kunststoff-Körpern mit Einzelkathetern oder äquidistant eingebetteten Kathetern oder Schläuchen für die Strahlentherapie, vorzugsweise Brachytherapie, nach der Afterloading-Methode,
dadurch gekennzeichnet,
daß die Katheter in der gelierten Paste des Kunststoff-Körpers in der Gelierform fest eingespannt sind und danach ausgeliert wird.

2. Verfahren gemäß Anspruch 1,
dadurch gekennzeichnet,
daß die Kunststoff-Formkörper aus Polyurethanen, Polyolefinen, Polycarbonaten, Polyvinychlorid, Polysulfonen, Polyacrylaten, Polyethern, Polyamiden, Silikonen und analogen Polymeren mit und ohne Weichmacher hergestellt werden.

3. Verfahren gemäß Anspruch 1,
dadurch gekennzeichnet,
daß die Katheter und ein gewebeverträgliches Gewebe in die Gelier- oder Aushärtform vorher oder in einem Zwischenschritt eingebracht werden.

4. Verfahren gemäß Anspruch 2,
dadurch gekennzeichnet,
daß im Falle höherer Gelier- oder Aushärttemperaturen, die für das Kathetermaterial schädlich sind, oberflächenvergütete Metallstifte mit Katheterpaßstücken eingesetzt und zur Einbringung der Katheter in den Kunststoff-Körper verwendet werden.

5. Verfahren gemäß Anspruch 1,
dadurch gekennzeichnet,
daß die Dichtflächen der Formteile konisch geformt sind.
